# FASCICULE DE BREVET EUROPEEN

(11) **EP 2 164 412 B1**
(45) Date de publication et mention de la délivrance du brevet: **03.09.2014**
(21) Numéro de dépôt: 08762708.9
(22) Date de dépôt: 21.05.2008
(51) Int. Cl.: A61B 17/70

(54) **IMPLANT VERTÉBRAL INTER-ÉPINEUX**
ZWISCHENWIRBELIMPLANTAT
INTER-SPINE VERTEBRAL IMPLANT

(30) Priorité: 22.05.2007 EP 07010112
(43) Date de publication de la demande: 24.03.2010
(73) Titulaire: Eden Spine Europe SA, 1207 Genève (CH)
(72) Inventeur: BEN-MOKHTAR, Mourad, CH-1207 Genève (CH); FUENTES, Jean-Marc, F-34790 Montpellier (FR)
(74) Mandataire: Micheli & Cie SA
(86) Numéro de dépôt international: PCT/IB2008/001266
(87) Numéro de publication internationale: WO 2008/142537

(56) Documents cités:
- WO-A-03/045262
- FR-A1- 2 709 245
- US-A1- 2002 143 331
- US-A1- 2006 036 240

## Description

La présente invention concerne un implant vertébral inter-épineux, c'est-à-dire un implant destiné à être inséré entre les apophyses épineuses de deux vertèbres adjacentes pour servir de stabilisateur et soulager notamment le disque intervertébral.

On connaît par la demande de brevet US 2002/0143331, figure 9, un implant vertébral inter-épineux comprenant des premier et second supports définissant des organes de fixation à des apophyses épineuses respectives adjacentes et entre lesquels est disposé un organe élastique de forme annulaire et coaxial avec les supports. L'organe élastique est disposé entre le fond d'une cavité annulaire du premier support et un organe mâle annulaire du second support qui est guidé axialement dans cette cavité. La paroi périphérique du premier support comprend une saillie annulaire interne qui peut coopérer avec un épaulement annulaire de l'organe mâle du second support pour limiter l'écartement des supports. La face d'extrémité de la paroi périphérique du premier support constitue également une butée qui peut coopérer avec un bord du second support pour limiter la compression de l'organe élastique.

Cet implant selon le document US 2002/0143331 présente plusieurs inconvénients. En premier lieu, on peut noter que cet implant et son organe élastique ont une forme circulaire. Pour obtenir une certaine capacité d'amortissement, l'organe élastique doit avoir des dimensions suffisantes. Or si l'on augmente le diamètre de l'implant, son encombrement dans la direction antéro-postérieure sera trop grand et nécessitera de délabrer dans une trop grande mesure les tissus vertébraux pour la mise en place de l'implant. D'autre part, la présence de l'organe mâle annulaire du second support et de la paroi périphérique du premier support limite de façon importante l'espace disponible pour l'organe élastique. Un autre inconvénient est que les butées qui limitent l'écartement des supports apparaissent sous-dimensionnées pour pouvoir véritablement résister à des charges physiologiques.

La demande de brevet FR 2 774 581, figure 4, décrit un implant vertébral interépineux comprenant les caractéristiques mentionnées dans le préambule de la revendication 1 annexée, à savoir :
- des premier et second supports destinés à être placés entre deux apophyses épineuses et définissant dans leur partie centrale des organes respectifs de fixation auxdites apophyses épineuses respectivement, ces organes de fixation étant situés dans un même plan sagittal, et
- des premier et second organes élastiquement compressibles dans la direction du rachis, ces organes élastiquement compressibles étant disposés entre les premier et second supports et de part et d'autre dudit plan sagittal.

Les organes élastiquement compressibles sont sous la forme de soufflets définissant des enceintes étanches remplies d'un fluide et d'un noyau en un matériau viscoélastique. Cet implant selon le document FR 2 774 581 semble présenter une plus grande capacité d'amortissement que celui décrit dans le document US 2002/0143331. Toutefois, cet implant apparaît difficilement réalisable car les extrémités des soufflets doivent être solidement fixées aux supports pour maintenir les enceintes étanches et éviter que les supports se détachent l'un de l'autre. Or le document FR 2 774 581 n'explique pas comment fixer les soufflets aux supports. Ce problème de fixation des soufflets aux supports est d'autant plus critique que l'implant ne comporte pas de butées pour limiter l'écartement des supports ni pour limiter la compression des soufflets. Il convient de noter de plus que les noyaux en matériau viscoélastique ne sont pas vraiment guidés et sont donc susceptibles de se déplacer latéralement ce qui peut déséquilibrer l'implant et perturber le fonctionnement des soufflets.

La présente invention vise à remédier aux inconvénients précités de l'état de la technique et propose à cet effet un implant vertébral inter-épineux comprenant les caractéristiques mentionnées plus haut en rapport avec le document FR 2 774 581 et caractérisé en ce qu'il comprend en outre des premier et second organes rigides de guidage autour desquels sont disposés respectivement les premier et second organes élastiquement compressibles, chacun de ces organes de guidage étant connecté à ses deux extrémités aux supports et comportant à au moins l'une de ses extrémités une butée conçue pour coopérer avec une butée du support correspondant pour limiter l'écartement des supports suivant la direction du rachis.

Ainsi, les organes rigides assurent à la fois une fonction de guidage et une fonction de butée. Cet agencement permet de garantir que les organes élastiques resteront bien en place dans l'implant et de conférer une grande robustesse aux butées. L'implant selon l'invention peut présenter un faible encombrement dans la direction antéro-postérieure, ce qui permet notamment de conserver une bonne partie des ligaments naturels. L'encombrement de l'implant dans la direction latérale est peu gênant car les tissus correspondants sont surtout constitués par du muscle.

Des modes de réalisation particuliers de l'invention sont définis dans les revendications dépendantes annexées 2 à 16.

D'autres caractéristiques et avantages de la présente invention apparaîtront à la lecture de la description détaillée suivante de plusieurs modes de réalisation de l'invention faite en référence aux dessins annexés dans lesquels :
- les figures 1 et 2 sont respectivement une vue en perspective et une vue en coupe frontale d'un implant vertébral inter-épineux selon un premier mode de réalisation ;
- les figures 3 et 4 sont des vues en coupe frontale d'un implant vertébral inter-épineux selon un second mode de réalisation, respectivement dans une position droite et dans une position inclinée ;
- la figure 5 est une vue en coupe frontale d'un implant vertébral inter-épineux selon un troisième mode de réalisation ;
- les figures 6 et 7 sont des vues en perspective respectivement d'un support à partie mâle et d'un support à partie femelle de l'implant vertébral inter-épineux selon le troisième mode de réalisation ;
- la figure 8 est une vue plane frontale montrant un implant selon l'invention en place entre deux apophyses épineuses ;
- les figures 9 et 10 sont respectivement une vue en perspective et une vue de profil d'une pièce d'arrêt utilisée pour arrêter un ligament servant au maintien de l'implant selon l'invention entre deux apophyses épineuses ; et
- la figure 11 est une vue en perspective de la pièce d'arrêt dans un état intermédiaire non plié.

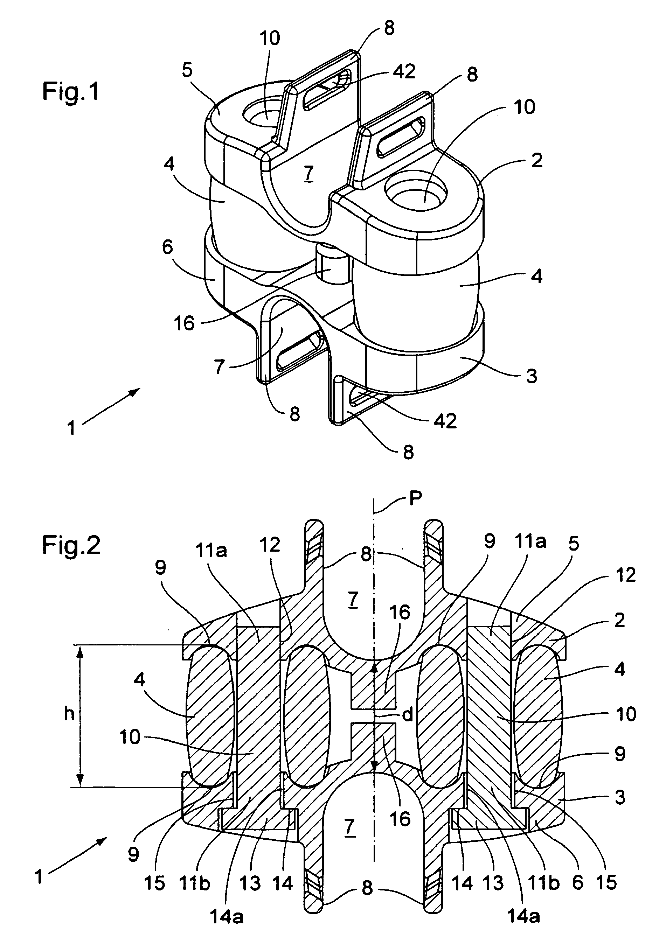

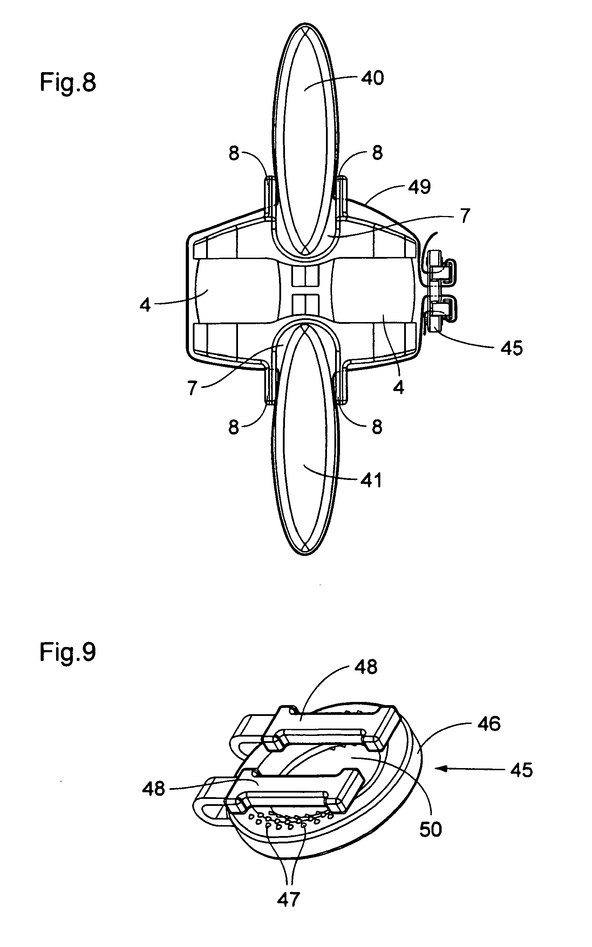

En référence aux figures 1 et 2, un implant vertébral inter-épineux 1 selon un premier mode de réalisation de l'invention comprend un support supérieur 2, un support inférieur 3 et deux organes élastiques 4 interposés entre les supports 2, 3. Chaque support 2, 3 comprend une platine 5, 6 allongée dans la direction latérale et dans la partie centrale de laquelle est prévu un évidement 7 en forme de U s'étendant dans la direction antéro-postérieure. Les parois latérales de l'évidement 7 sont prolongées par une paire d'ailettes 8 qui fait saillie sur la surface extérieure de la platine 5, 6. La paire d'ailettes 8 forme avec l'évidement 7 un organe de fixation à une apophyse épineuse. Plus précisément, l'évidement 7 et l'espace entre les ailettes 8 sont destinés à recevoir une apophyse épineuse, le maintien de cette dernière dans l'organe 7, 8 étant assuré par exemple par un ou des ligaments synthétiques comme cela sera expliqué plus loin en relation avec la figure 8. Les organes de fixation respectifs 7, 8 des supports 2, 3 sont situés dans un même plan sagittal P, qui constitue un plan de symétrie pour l'implant 1. Chaque support 2, 3, avec sa platine 5, 6 et ses ailettes 8, est typiquement une pièce monobloc réalisée en un matériau biocompatible, par exemple métallique, de plus grande dureté que celle des organes élastiques 4.

Les organes élastiques 4 sont alignés dans la direction latérale et sont situés de part et d'autre du plan sagittal de symétrie P. Les organes élastiques 4 sont élastiquement compressibles dans la direction du rachis (direction verticale P sur la figure 2) et sont typiquement réalisés en une matière viscoélastique telle que le silicone. Les organes élastiques 4 ont une forme générale cylindrique annulaire et leurs extrémités reposent, sans y être fixées par un autre moyen, dans des creusures annulaires 9 pratiquées dans les faces intérieures en regard des platines 5, 6. Chaque organe élastique 4 entoure une tige de guidage rigide 10, typiquement métallique, qui s'étend parallèlement au plan P, dans la direction du rachis, et qui est connectée à ses deux extrémités aux platines 5, 6 des supports 2, 3, respectivement. Chaque tige de guidage 10 est plus précisément sous la forme d'une vis dont l'extrémité filetée 11a est vissée dans un alésage taraudé correspondant 12 de la platine 5 du support supérieur 2 et dont la tête 13 s'appuie contre un épaulement annulaire 14 dans un alésage traversant 15 de la platine 6 du support inférieur 3 dans lequel passe l'autre extrémité 11 b de la tige de la vis 10. Chaque tige de guidage 10 est libre par rapport à la paroi de l'alésage 15 correspondant de sorte que le support 3 est mobile axialement, c'est-à-dire dans la direction du rachis, par rapport au support 2. La tête 13 des tiges de guidage 10 et les épaulements 14 constituent toutefois des butées qui limitent de manière fiable l'écartement des supports 2, 3 et empêchent ainsi ces derniers de se séparer pendant les mouvements de flexion du patient. Les organes élastiques 4 sont ainsi maintenus en permanence bien en place autour des tiges de guidage 4.

De préférence, la section de chaque alésage 15 est plus grande que celle de la tige de guidage 10 correspondante afin de ménager un jeu 14a entre les parois des alésages 15 et les tiges de guidage 10 pour autoriser des mouvements d'inclinaison des supports 2, 3 l'un par rapport à l'autre dans le plan frontal (mouvements de flexion latérale du patient) ou dans le plan sagittal (mouvements de flexion / extension du patient) ainsi que des mouvements de rotation axiale des supports 2, 3 l'un par rapport à l'autre (torsion), tous ces mouvements ayant une amplitude limitée par les parois des alésages 15.

De préférence, les tiges de guidage 10 sont vissées de manière à rapprocher suffisamment les supports 2, 3 pour que les organes élastiques 4 soient dans un état de précontrainte avant la mise en place de l'implant. Une telle précontrainte permet à l'implant de supporter des charges jusqu'à un certain seuil sans compression supplémentaire des organes élastiques 4. Il est notamment possible de choisir l'état de précontrainte de telle sorte qu'une fois l'implant mis en place, il soit capable de supporter les charges exercées par le rachis, lorsque le patient est dans la station érigée, sans compression supplémentaire des organes élastiques 4. Ceci permet de garantir un maintien efficace de l'écartement des apophyses épineuses.

Dans des variantes, les extrémités 11a des tiges de guidage 10 pourraient être fixées dans le support 2 d'une autre manière que par vissage, par exemple par chassage, collage ou soudage. Par le terme « fixées » on entend ici une fixation qui maintient les extrémités 11a immobiles axialement par rapport au support 2 pendant des mouvements relatifs des supports 2, 3.

L'implant 1 comprend en outre, de préférence, deux plots 16 qui font saillie en regard l'un de l'autre sur les faces intérieures respectives des platines 5, 6, dans la partie centrale de l'implant 1, entre les organes élastiques 4. Ces plots 16 peuvent venir en butée l'un contre l'autre pendant des mouvements d'extension du patient pour limiter la compression des organes élastiques 4 et protéger ainsi ces derniers.

Comme on peut le voir à la figure 2, l'espace entre les organes élastiques 4 peut être utilisé pour rapprocher l'organe de fixation 7, 8 du support supérieur 2 de l'organe de fixation 7, 8 du support inférieur 3. En effet, un inconvénient des implants inter-épineux traditionnels est qu'ils nécessitent de réséquer une partie des apophyses épineuses pour dégager un espace suffisant permettant l'insertion de l'implant. Une telle résection, outre son caractère fastidieux, peut entraîner une fragilité puis une rupture des apophyses épineuses. Avec l'implant selon l'invention, une telle résection n'est pas nécessaire, ou peut être effectuée de manière minime, grâce aux évidements 7 de réception des apophyses épineuses qui sont pratiqués dans les platines 5, 6 et qui sont proches l'un de l'autre. De préférence, la distance d entre les fonds respectifs des évidements 7 est sensiblement égale voire inférieure à la hauteur h des organes élastiques 4. Ainsi, l'implant peut être mis en place sans résection des apophyses épineuses tout en comportant des organes élastiques 4 de grande hauteur et de grande capacité d'amortissement.

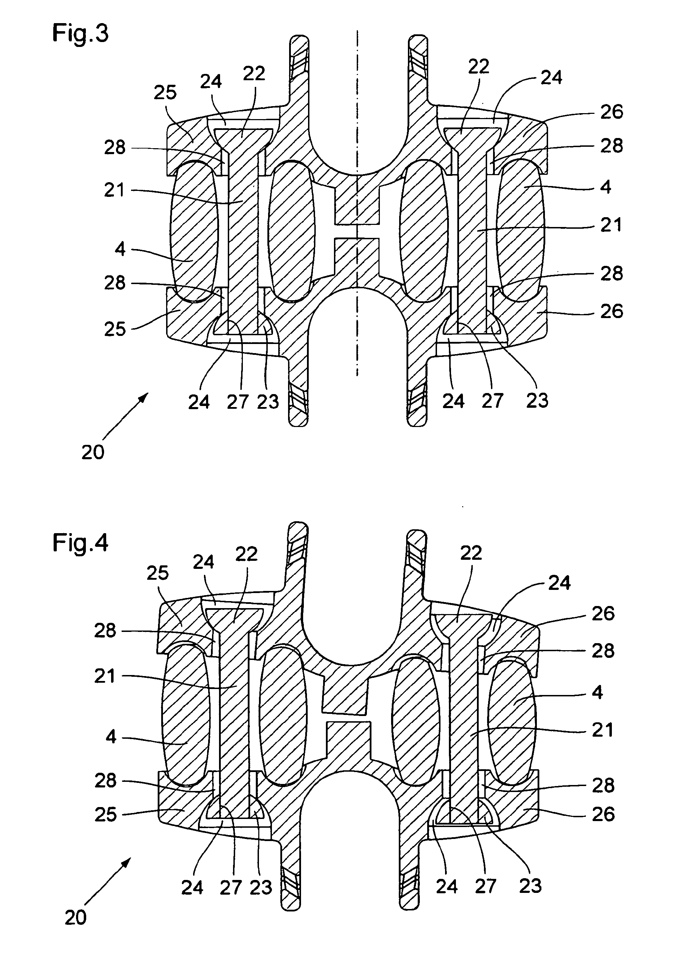

Les figures 3 et 4 montrent un implant vertébral inter-épineux 20 selon un second mode de réalisation de l'invention. Cet implant 20 diffère de l'implant 1 selon le premier mode de réalisation en ce que les tiges de guidage 21 ne sont pas fixées dans l'une des platines mais comportent à chacune de leurs extrémités une tête de forme hémisphérique 22, 23. Ces têtes hémisphériques 22, 23 reposent dans des logements de forme hémisphérique 24 mais de plus grand rayon pratiqués dans les platines 25 des supports 26. Les deux têtes 22, 23 de chaque tige de guidage 21 jouent le rôle de butées pour limiter l'écartement des supports 26. L'une au moins de ces têtes 22, 23 est un écrou 23 qui coopère avec un filetage 27 de la tige 21 pour régler l'écartement des supports 26 et donc la précontrainte des organes élastiques 4. Des jeux 28 sont ménagés entre les tiges de guidage 21 et les parois des alésages des platines 25 dans lesquels passent les tiges 21 pour permettre aux supports 26 de s'incliner l'un par rapport à l'autre dans le plan frontal pendant un mouvement de flexion latérale du patient (comme montré à la figure 4) ou dans le plan sagittal pendant un mouvement de flexion / extension du patient ou de pivoter axialement l'un par rapport à l'autre lors d'un mouvement de torsion du patient. La forme sphérique des têtes 22, 23 et des logements 24 facilite lesdits mouvements d'inclinaison.

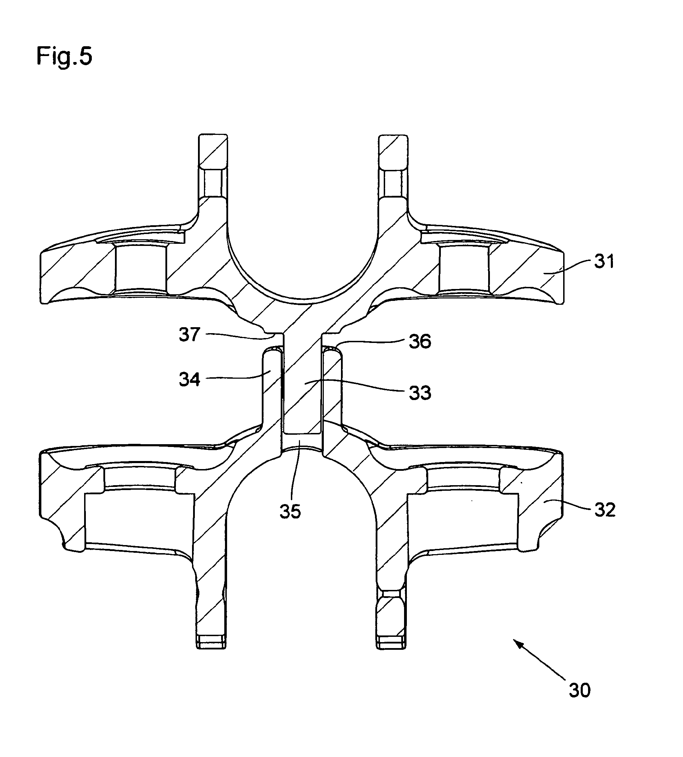

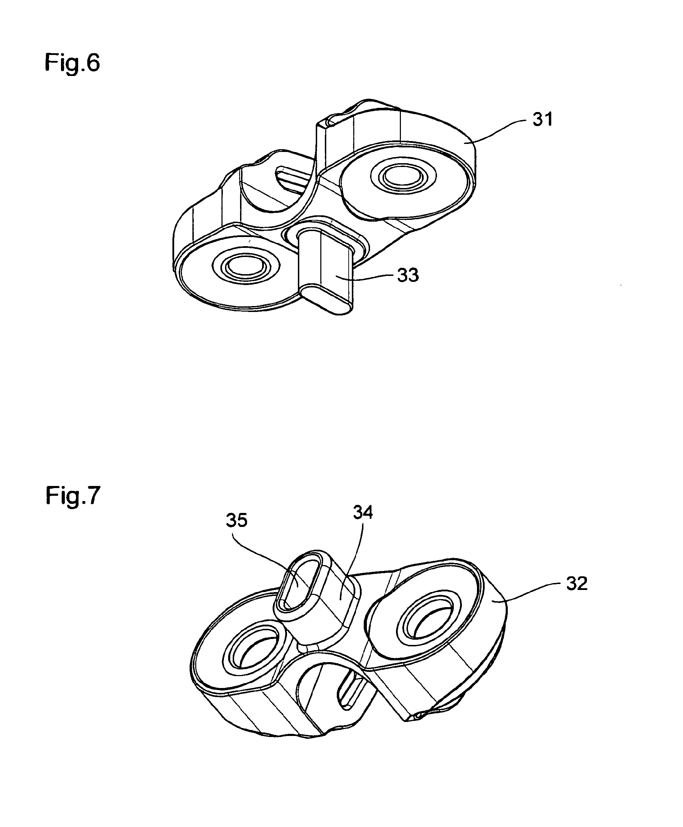

Les figures 5 à 7 montrent un implant intervertébral inter-épineux 30 selon un troisième mode de réalisation. L'implant 30 comprend deux supports 31, 32 du même type que les supports 2, 3 du premier mode de réalisation ou que les supports 26 du second mode de réalisation, des tiges de guidage (non représentées) du même type que les tiges 10 du premier mode de réalisation ou que les tiges 21 du second mode de réalisation, et des organes élastiques (non représentés) du même type que les organes élastiques 4 des premier et second modes de réalisation. A la place des simples plots de butée 16, l'implant 30 comprend, dans sa partie centrale située entre les organes élastiques, un assemblage d'une partie mâle 33 faisant saillie depuis la face intérieure de la platine du support 31 et d'une partie femelle 34 faisant saillie depuis la face intérieure de la platine de l'autre support 32. La partie femelle 34 comporte un alésage 35 dans lequel coulisse la partie mâle 33 lors de déplacements relatifs axiaux des supports 31, 32. Une surface d'extrémité 36 de la partie femelle 34 peut buter contre une surface d'appui 37 du support 31 entourant la base de la partie mâle 33 pendant des mouvements d'extension du patient pour limiter la compression des organes élastiques et protéger ainsi ces derniers. La section de l'alésage 35 est plus grande que la section de la partie mâle 33 pour autoriser des mouvements relatifs d'inclinaison dans le plan frontal et dans le plan sagittal des supports 31, 32 dans des limites déterminées. La partie mâle 33 et l'alésage 35 ont tous les deux une section de forme non circulaire, à savoir oblongue dans l'exemple représenté, pour limiter les mouvements relatifs de rotation axiale des supports 31, 32. Le jeu entre les parties mâle 33 et femelle 34 est moins grand que celui entre les tiges de guidage et les supports 31, 32. Ainsi, c'est l'assemblage 33, 34 qui limite les mouvements relatifs d'inclinaison et de rotation axiale des supports 31, 32 et non plus l'assemblage des tiges de guidage et des supports 31, 32. Cet agencement permet de protéger les tiges de guidage.

La figure 8 montre comment l'implant selon l'invention peut être mis en place. A la figure 8, l'implant représenté est l'implant 1 ou 20. Il pourrait toutefois être l'implant 30. L'implant est placé entre deux apophyses épineuses consécutives 40, 41 au besoin en comprimant les organes élastiques 4 pour faciliter l'insertion de l'implant puis en relâchant l'effort de compression une fois l'implant en place. Les ailettes 8 comprennent chacune une fente 42 (cf. figure 1) s'étendant dans la direction antéro-postérieure. Ces fentes 42 permettent le passage d'un ou plusieurs ligaments synthétiques servant à maintenir de manière souple les apophyses épineuses 40, 41 dans les organes de fixation 7, 8. En fonction de l'état de précontrainte des organes élastiques 4, ces ligaments synthétiques sont plus ou moins sollicités pendant des mouvements de flexion du patient. Par leur élasticité, ces ligaments synthétiques autorisent un écartement des vertèbres même lorsque l'écartement des supports 2, 3, respectivement 26, est bloqué par les butées 13, 14, respectivement 22, 23, 24. Cet écartement des vertèbres reste toutefois dans des limites physiologiques.

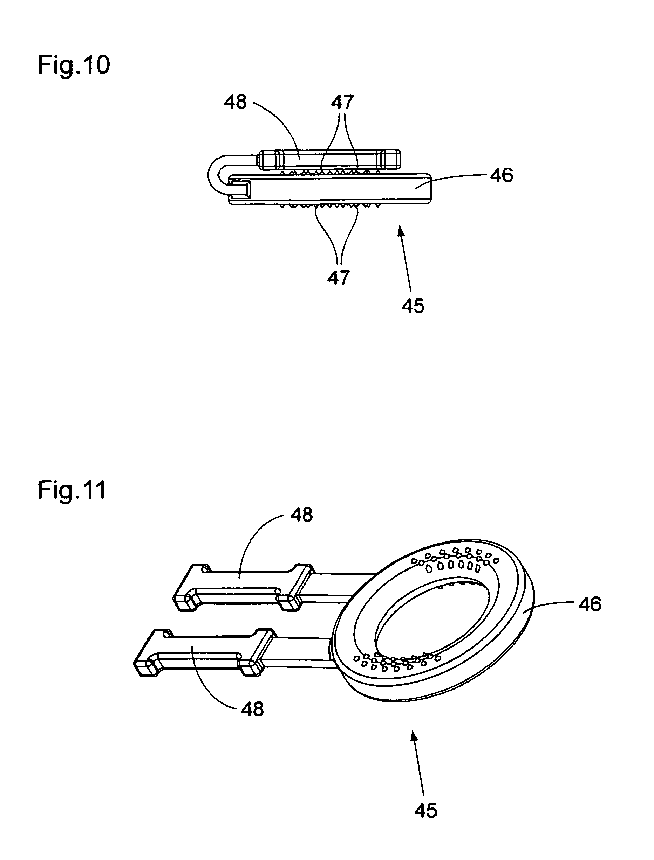

Traditionnellement, de tels ligaments synthétiques sont arrêtés et unis les uns aux autres au moyen de noeuds. Selon une caractéristique de l'invention, une pièce 45 est utilisée pour arrêter et unir les parties d'extrémité d'un ligament sans faire de noeud. Cette pièce est représentée aux figures 9 et 10. Elle comprend une partie annulaire 46 comportant des picots 47 sur ses surfaces supérieure et inférieure et deux languettes parallèles 48 rattachées à l'une de leurs extrémités à la surface périphérique externe de la partie annulaire 46 et ramenées au-dessus de la partie annulaire 46 par un pliage de 180°. La figure 11 montre la pièce d'arrêt 45 avant le pliage des languettes 48.

Comme représenté à la figure 8, la pièce d'arrêt 45 est placée libre à côté de l'implant avec sa face inférieure tournée vers l'implant. Un ligament synthétique 49 fait le tour de l'implant sensiblement dans le plan frontal en passant par les fentes 42 des ailettes 8 et en contournant l'apophyse épineuse supérieure 40 par son extrémité supérieure et l'apophyse épineuse inférieure 41 par son extrémité inférieure. Les deux bouts du ligament 49 passent dans la partie centrale de l'ouverture 50 de la partie annulaire 46 par la face inférieure de cette dernière, puis forment une boucle autour des languettes 48 respectivement pour retraverser l'ouverture 50 dans la partie périphérique de celle-ci. Les extrémités du ligament 49 sont laissées libres. En tirant sur ces extrémités, le chirurgien serre le ligament 49 dans la pièce d'arrêt 45. Les picots 47 coopèrent alors avec le ligament 49 pour assurer un bon maintien de ce dernier.

## Revendications

1. Implant vertébral inter-épineux comprenant :
- des premier et second supports (2, 3) destinés à être placés entre deux apophyses épineuses et définissant dans leur partie centrale des organes respectifs (7, 8) de fixation auxdites apophyses épineuses respectivement, ces organes de fixation (7, 8) étant situés dans un même plan sagittal (P),
- des premier et second organes élastiquement compressibles (4) dans la direction du rachis, ces organes élastiquement compressibles (4) étant disposés entre les premier et second supports (2, 3) et de part et d'autre dudit plan sagittal (P),
**caractérisé en ce qu'**il comprend en outre des premier et second organes rigides de guidage (10) autour desquels sont disposés respectivement les premier et second organes élastiquement compressibles (4), chacun de ces organes de guidage (10) étant connecté à ses deux extrémités aux supports (2, 3) et comportant à au moins l'une de ses extrémités une butée (13) conçue pour coopérer avec une butée (14) du support correspondant (3) pour limiter l'écartement des supports (2, 3) suivant la direction du rachis.

2. Implant selon la revendication 1, **caractérisé en ce que** les butées (13) des organes de guidage (10) maintiennent les organes élastiquement compressibles (4) dans un état précontraint.

3. Implant selon la revendication 1 ou 2, **caractérisé en ce que** les organes élastiquement compressibles (4) sont en un matériau viscoélastique.

4. Implant selon l'une quelconque des revendications 1 à 3, **caractérisé en ce qu'**il comprend un jeu (14a) entre chaque organe de guidage (10) et l'un au moins des supports (2, 3) pour autoriser des mouvements relatifs d'inclinaison et de rotation axiale des supports (2, 3).

5. Implant selon la revendication 4, **caractérisé en ce que** les supports (31, 32) définissent respectivement, dans une zone centrale située entre les organes élastiquement compressibles, une partie femelle (34) comportant un alésage (35) et une partie mâle (33) assemblée à la partie femelle (34) pour coulisser axialement dans l'alésage (35) avec un jeu permettant des mouvements relatifs d'inclinaison des supports (31, 32), la section de la partie mâle (33) et de l'alésage (35) ayant une forme non circulaire pour limiter des mouvements relatifs de rotation axiale des supports (31, 32), ledit jeu étant moins grand que le jeu (14a) entre les organes de guidage et les supports (31, 32) afin que lesdits mouvements relatifs d'inclinaison et de rotation axiale soient limités par l'assemblage des pièces mâle et femelle (33, 34) et non pas par l'assemblage des organes de guidage et des supports (31, 32).

6. Implant selon l'une quelconque des revendications 1 à 5, **caractérisé en ce que** les organes de guidage (10) sont fixés à l'une de leurs extrémités (11) dans l'un (2) des supports (2, 3) et comportent une butée (13) à l'autre extrémité.

7. Implant selon la revendication 4 ou 5, **caractérisé en ce que** l'une au moins des extrémités (22, 23) de chaque organe de guidage (21) a une forme sphérique favorisant une inclinaison des supports (26) l'un par rapport à l'autre.

8. Implant selon la revendication 7, **caractérisé en ce que** l'extrémité de forme sphérique ou l'une au moins des extrémités de forme sphérique de chaque organe de guidage (21) comprend un écrou (23) de forme sphérique.

9. Implant selon l'une quelconque des revendications 1 à 8, **caractérisé en ce que** les supports (2, 3) définissent en outre des butées respectives (16 ; 36, 37) situées entre les organes élastiquement compressibles (4) et pouvant venir en contact l'une avec l'autre pour limiter la compression des organes élastiquement compressibles (4).

10. Implant selon l'une quelconque des revendications 1 à 9, **caractérisé en ce que** les supports (2, 3) sont sous la forme de platines (5, 6) destinées à être placées entre des apophyses épineuses et comportant lesdits organes de fixation (7, 8).

11. Implant selon l'une quelconque des revendications 1 à 10, **caractérisé en ce que** lesdits organes de fixation (7, 8) définissent chacun un logement (7) destiné à recevoir une apophyse épineuse.

12. Implant selon la revendication 11, **caractérisé en ce que** la distance (d) entre les fonds respectifs des logements (7) est sensiblement égale ou inférieure à la hauteur (h) des organes élastiquement compressibles (4).

13. Implant selon la revendication 12, **caractérisé en ce que** ladite distance (d) est inférieure à ladite hauteur (h)

14. Implant selon l'une quelconque des revendications 1 à 13, **caractérisé en ce que** les organes de fixation (7, 8) comprennent chacun des ailettes (8) entre lesquelles peut être reçue une apophyse épineuse, ces ailettes (8) comprenant chacune une ouverture (42) permettant le passage d'un ligament.

15. Dispositif comprenant un implant selon l'une quelconque des revendications 1 à 14 et une pièce d'arrêt (45) destinée à rester libre à proximité de l'implant et adaptée pour arrêter sans noeud des parties d'extrémité d'un ligament (49) de maintien de l'implant entre les apophyses épineuses.

16. Dispositif selon la revendication 15, **caractérisé en ce que** la pièce d'arrêt (45) comprend une partie annulaire (46) et des languettes parallèles (48) repliées par-dessus la partie annulaire (46).

## Patentansprüche

1. Interspinales Wirbelsäulenimplantat, umfassend:
- erste und zweite Träger (2, 3), die dazu gedacht sind, zwischen zwei Dornfortsätzen eingesetzt zu werden und in ihrem mittleren Teil jeweilige Elemente (7, 8) zur Befestigung jeweils an den Dornfortsätzen definieren, wobei sich diese Befestigungselemente (7, 8) in der gleichen Sagittalebene (P) befinden,
- erste und zweite Elemente (4), die in Richtung der Wirbelsäule elastisch zusammendrückbar sind, wobei diese elastisch zusammendrückbaren Elemente (4) zwischen den ersten und zweiten Trägern (2, 3) auf beiden Seiten der Sagittalebene (P) angeordnet sind,
**dadurch gekennzeichnet, dass** es ferner erste und zweite starre Führungselemente (10) umfasst, um die jeweils die ersten und zweiten elastisch zusammendrückbaren Elemente (4) angeordnet sind, wobei jedes dieser Führungselemente (10) an seinen beiden Enden mit den Trägern (2, 3) verbunden ist und an mindestens einem seiner Enden einen Anschlag (13) umfasst, der ausgelegt ist, um mit einem Anschlag (14) des entsprechenden Trägers (3) zusammenzuwirken, um den Zwischenraum der Träger (2, 3) in Richtung der Wirbelsäule einzuschränken.

2. Implantat nach Anspruch 1, **dadurch gekennzeichnet, dass** die Anschläge (13) der Führungselemente (10) die elastisch zusammendrückbaren Elemente (4) in einem vorgespannten Zustand halten.

3. Implantat nach Anspruch 1 oder 2, **dadurch gekennzeichnet, dass** die elastisch zusammendrückbaren Elemente (4) aus einem viskoelastischen Material bestehen.

4. Implantat nach einem der Ansprüche 1 bis 3, **dadurch gekennzeichnet, dass** es einen Spielraum (14a) zwischen jedem Führungselement (10) und mindestens einem der Träger (2, 3) umfasst, um relative Neigungs- und Axialdrehbewegungen der Träger (2, 3) zuzulassen.

5. Implantat nach Anspruch 4, **dadurch gekennzeichnet, dass** die Träger (31, 32) jeweils in einer mittleren Zone, die sich zwischen den elastisch zusammendrückbaren Elementen befindet, einen inneren Teil (34), der eine Bohrung (35) umfasst, und einen äußeren Teil (33), der mit dem inneren Teil (34) zusammengefügt ist, um in der Bohrung (35) mit einem Spielraum, der relative Neigungsbewegungen der Träger (31, 32) zulässt, axial zu gleiten, definieren, wobei der Querschnitt des äußeren Teils (33) und der Bohrung (35) eine nicht kreisförmige Form aufweist, um relative Axialdrehbewegungen der Träger (31, 32) einzuschränken, wobei der Spielraum weniger groß ist als der Spielraum (14a) zwischen den Führungselementen und den Trägern (31, 32), damit die relativen Neigungs- und Axialdrehbewegungen durch das Zusammenfügen der äußeren und inneren Teile (33, 34) und nicht durch das Zusammenfügen der Führungselemente und der Träger (31, 32) eingeschränkt werden.

6. Implantat nach einem der Ansprüche 1 bis 5, **dadurch gekennzeichnet, dass** die Führungselemente (10) an einem ihrer Enden (11) in einem (2) der Träger (2, 3) befestigt sind und einen Anschlag (13) an dem anderen Ende umfassen.

7. Implantat nach Anspruch 4 oder 5, **dadurch gekennzeichnet, dass** mindestens eines der Enden (22, 23) jedes Führungselements (21) eine Kugelform aufweist, die eine Neigung der Träger (26) im Verhältnis zueinander begünstigt.

8. Implantat nach Anspruch 7, **dadurch gekennzeichnet, dass** das kugelförmige Ende oder mindestens eines der kugelförmigen Enden jedes Führungselements (21) eine kugelförmige Mutter (23) umfasst.

9. Implantat nach einem der Ansprüche 1 bis 8, **dadurch gekennzeichnet, dass** die Träger (2, 3) ferner jeweilige Anschläge (16; 36, 37) umfassen, die sich zwischen den elastisch zusammendrückbaren Elementen (4) befinden und einander berühren können, um das Zusammendrücken der elastisch zusammendrückbaren Elemente (4) einzuschränken.

10. Implantat nach einem der Ansprüche 1 bis 9, **dadurch gekennzeichnet, dass** die Träger (2, 3) in Form von Platten (5, 6) vorliegen, die dazu gedacht sind, zwischen Dornfortsätzen eingesetzt zu werden, und die Befestigungselemente (7, 8) umfassen.

11. Implantat nach einem der Ansprüche 1 bis 10, **dadurch gekennzeichnet, dass** die Befestigungselemente (7, 8) jeweils eine Aufnahme (7) definieren, die dazu gedacht ist, einen Dornfortsatz aufzunehmen.

12. Implantat nach Anspruch 11, **dadurch gekennzeichnet, dass** der Abstand (d) zwischen den jeweiligen Böden der Aufnahmen (7) im Wesentlichen gleich oder kleiner als die Höhe (h) der elastisch zusammendrückbaren Elemente (4) ist.

13. Implantat nach Anspruch 12, **dadurch gekennzeichnet, dass** der Abstand (d) kleiner als die Höhe (h) ist.

14. Implantat nach einem der Ansprüche 1 bis 13, **dadurch gekennzeichnet, dass** die Befestigungselemente (7, 8) jeweils Stege (8) umfassen, zwischen denen ein Dornfortsatz aufgenommen werden kann, wobei diese Stege (8) jeweils eine Öffnung (42) umfassen, die den Durchgang eines Bandes ermöglichen.

15. Vorrichtung, umfassend ein Implantat nach einem der Ansprüche 1 bis 14 und ein Sicherungsstück (45), das dazu gedacht ist, in der Nähe des Implantats frei zu bleiben, und geeignet ist, um ohne Knoten Endteile eines Bandes (49) zum Halten des Implantats zwischen den Dornfortsätzen zu sichern.

16. Vorrichtung nach Anspruch 15, **dadurch gekennzeichnet, dass** das Sicherungsstück (45) einen ringförmigen Teil (46) und parallele Laschen (48), die über dem ringförmigen Teil (46) umgebogen sind, umfasst.

## Claims

1. An interspinous vertebral implant comprising:
- first and second supports (2, 3) intended to be placed between two spinal processes and defining in their central part respective members (7, 8) for fixation to said spinal processes respectively, these fixation members (7, 8) being located in a same sagittal plane (P);
- first and second members (4) elastically compressible in the direction of the spine, these elastically compressible members (4) being arranged between the first and second supports (2, 3) and on both sides of said sagittal plane (P);
**characterized by** further comprising first and second rigid guiding members (10) around which the first and second elastically compressible members (4) are respectively arranged, each of these guiding members (10) being connected at its two ends to the supports (2, 3) and comprising at at least one of its ends an abutment (13) designed to cooperate with an abutment (14) of the corresponding support (3) to limit the spacing of the supports (2, 3) in the direction of the spine.

2. The implant according to claim 1, **characterized in that** the abutments (13) of the guiding members (10) maintain the elastically compressible members (4) in a prestrained state.

3. The implant according to claim 1 or 2, **characterized in that** the elastically compressible members (4) are made of a visco-elastic material.

4. The implant according to any one of claims 1 to 3, **characterized by** comprising a play (14a) between each guiding member (10) and at least one of the supports (2, 3) to allow relative inclination and axial rotation movements of the supports (2, 3).

5. The implant according to claim 4, **characterized in that** the supports (31, 32) respectively define, in a central zone located between the elastically compressible members, a female part (34) comprising a bore (35) and a male part (33) assembled to the female part (34) to slide axially in the bore (35) with a play allowing relative inclination movements of the supports (31, 32), the section of the male part (33) and of the bore (35) having a non-circular shape to limit relative axial rotation movements of the supports (31, 32), said play being smaller than the play (14a) between the guiding members and the supports (31, 32) such that said relative inclination and axial rotation movements are limited by the assembly of the male and female parts (33, 34) and not by the assembly of the guiding members and of the supports (31, 32).

6. The implant according to any one of claims 1 to 5, **characterized in that** the guiding members (10) are fixed at one of their ends (11) in one (2) of the supports (2, 3) and comprise an abutment (13) at the other end.

7. The implant according to claim 4 or 5, **characterized in that** at least one of the ends (22, 23) of each guiding member (21) has a spherical shape favoring an inclination of the supports (26) relative to one another.

8. The implant according to claim 7, **characterized in that** the spherical shaped end or at least one of the spherical shaped ends of each guiding member (21) comprises a screw nut (23) of spherical shape.

9. The implant according to any one of claims 1 to 8, **characterized in that** the supports (2, 3) further define respective abutments (16; 36, 37) which are located between the elastically compressible members (4) and are able to come in contact with each other to limit the compression of the elastically compressible members (4).

10. The implant according to any one of claims 1 to 9, **characterized in that** the supports (2, 3) are in the shape of plates (5, 6) intended to be placed between spinal processes and comprising said fixation members (7, 8).

11. The implant according to any one of claims 1 to 10, **characterized in that** said fixation members (7, 8) each define a recess (7) intended to receive a spinal process.

12. The implant according to claim 11, **characterized in that** the distance (d) between the respective bottoms of the recesses (7) is substantially equal to or lower than the height (h) of the elastically compressible members (4).

13. The implant according to claim 12, **characterized in that** said distance (d) is lower than said height (h).

14. The implant according to any one of claims 1 to 13, **characterized in that** the fixation members (7, 8) each comprise fins (8) between which a spinal process can be received, these fins (8) each comprising an aperture (42) allowing the passage of a ligament.

15. A device comprising an implant according to any one of claims 1 to 14 and a stop part (45) intended to remain free near the implant and adapted to stop without knotting end parts of a ligament (49) for maintaining of the implant between the spinal processes.

16. The device according claim 15, **characterized in that** the stop part (45) comprises an annular part (46) and parallel strips (48) folded over the annular part (46).
